Europäisches Patentamt

European Patent Office

Office européen des brevets

(19)

(11) Veröffentlichungsnummer: **0 125 400**
**B1**

(12) # EUROPÄISCHE PATENTSCHRIFT

(45) Veröffentlichungstag der Patentschrift:
01.07.87

(51) Int. Cl.⁴: **A 61 F 2/52**

(21) Anmeldenummer: **84101858.3**

(22) Anmeldetag: **22.02.84**

(54) **Brustprothese.**

(30) Priorität: **18.04.83 DE 8311438 U**

(43) Veröffentlichungstag der Anmeldung:
**21.11.84 Patentblatt 84/47**

(45) Bekanntmachung des Hinweises auf die Patenterteilung:
**01.07.87 Patentblatt 87/27**

(84) Benannte Vertragsstaaten:
**AT BE CH DE FR GB IT LI LU NL SE**

(56) Entgegenhaltungen:
**DE-A-3 046 785**
**FR-A-2 451 738**
**GB-A-1 110 479**
**US-A-3 366 975**
**US-A-3 641 592**
**US-A-3 811 133**

(73) Patentinhaber: **Tertulin, Eberl, Nonnenwaldstrasse 25, D-8122 Penzberg (DE)**
Patentinhaber: **Anita- Spezialmiederfabrik Dr. Helbig & Co., Oskar- Pirlo- Strasse 23, A-6332 Kufstein (AT)**

(72) Erfinder: **Eberl, Tertulin, Nonnenwaldstrasse 25, D-8122 Penzberg (DE)**
Erfinder: **Weber- Unger, Georg, Pienzenauerstrasse 15, A-6330 Kufstein (AT)**

(74) Vertreter: **Haug, Dietmar, Patentanwälte Andrae, Flach, Haug Steinstrasse 44, D-8000 München 80 (DE)**

## Beschreibung

Die Erfindung betrifft eine Brustprothese gemäß dem Oberbegriff des Patentanspruches 1.

Eine solche Brustprothese ist aus der FR-A-2 451 738 bekannt. Bei dieser Brustprothese hat der Prothesenkörper, der ganz aus Silikonkautschuk besteht, auf seiner Rückseite eine pilzförmige Vertiefung, in deren Kopfteil ein Polsterkörper aus einem mit Gas oder Flüssigkeit gefüllten Beutel angeordnet ist. Der Polsterkörper wird in dem Kopfteil der Vertiefung durch einen sich radial einwärts erstreckenden rückwärtigen Wandabschnitt des Prothesenkörpers, in dessen Mitte sich der einen Kanal bildende Fußteil der Vertiefung befindet, fest gehalten. Durch die Vertiefung und die Verwendung eines Polsterkörpers, der ein geringeres spezifisches Gewicht als der Prothesenkörper hat, verringert sich das Gesamtgewicht der vorbekannten Brustprothese etwas gegenüber dem einer massiven, dem Volumen und dem Gewicht der entfernten Brust entsprechenden Vollprothese, die von den Trägerinnen oftmals als zu schwer empfunden werden, wobei der Polsterkörper aufgrund seiner Stützwirkung beim Tragen der Prothese in einem Büstenhalter ein Zusammensacken des Prothesenkörpers verhindert. Aufgrund des relativ großen Volumens des Prothesenkörpers hat die vorbekannte Brustprothese aber immer noch ein so hohes Gewicht, daß sie die Trägerinnen schwerer als die entfernte Brust empfinden. Die vorbekannte Brustprothese ist dafür bestimmt, in einem Büstenhalter getragen zu werden, wobei die ebene Außenfläche des sich radial einwärts erstreckenden Wandabschnitts des Prothesenkörpers unmittelbar am Körper der Trägerin anliegt, wodurch Druckstellen auf der Operationswunde oder -narbe entstehen können, insbesondere dann, wenn die Brust nicht vollständig entfernt worden ist.

Eine Brustprothese mit einem dem Prothesenkörper der gattungsgemäßen Brustprothese entsprechenden Prothesenkörper ist aus der DE-A-3 046 785 bekannt. Diese Brustprothese ist aber dafür bestimmt, mittels eines Formteiles, das in eine Vertiefung auf der Rückseite des Prothesenkörpers eingesetzt ist, direkt am Körper der Trägerin befestigt zu werden. Das Formteil besteht vorzugsweise aus demselben Material wie der Prothesenkörper, wodurch sich ein der entfernten Brust entsprechendes Gesamtgewicht der Prothese ergibt. Die Vertiefung ist rotationssymmetrisch mit einem relativ kleinen Durchmesser und relativ flach mit einem ebenen Boden ausgebildet und koaxial in bezug auf die senkrechte Mittelachse des Prothesenkörpers angeordnet. Die seitliche Wand der Vertiefung besteht aus einem an den hinteren ebenen Randflächenabschnitt des Prothesenkörpers angrenzenden, ringförmigen, radial nach innen vorspringenden, einen Wulst bildenden Abschnitt und einem an den ebenen Boden der Vertiefung angrenzenden, eine Ringnut bildenden Abschnitt, der einen größeren Durchmesser als der den Wulst bildende Wandabschnitt hat. Das Formteil hat eine der Form der Vertiefung angepaßte Oberfläche und ist formschlüssig in der Vertiefung angeordnet, wobei es von dem wulstförmigen Wandabschnitt in der Vertiefung lösbar gehalten wird und relativ zu sich eine Drehung des Prothesenkörpers um dessen senkrechte Mittelachse gestattet. Die dem Körper der Prothesenträgerin zugewandte Seite des Formteiles ist eben und bündig zu dem hinteren Randflächenabschnitt des Prothesenkörpers und weist eine Haftklebeschicht auf, mit der die Prothese direkt am Körper der Prothesenträgerin zu befestigen ist. Bei dieser vorbekannten Brustprothese ist somit auch noch nach ihrer Befestigung am Körper der Trägerin eine Drehung des Prothesenkörpers zur Einstellung der richtigen Sitzposition der Prothese möglich. Aufgrund der flachen Form und der geringen Größe der Vertiefung wird die dem Prothesenkörper eigene Formstabilität durch die Vertiefung nur wenig beeinträchtigt, so daß das Formteil nur eine entsprechend geringe Stützwirkung auf den Prothesenkörper ausüben muß. Die vorbekannte Brustprothese kann nur von solchen brustamputierten Frauen verwendet werden, bei denen die Brust vollständig unter Bildung einer ebenen Auflagefläche für die Prothese entfernt worden ist und keine Reizung der Haut an der Auflagefläche infolge des Anheftens der Prothese am Körper auftritt.

Andere vorbekannte Brustprothesen, die leichter als Vollprothesen sind, aber auch aus einem eine Konsistenz und ein spezifisches Gewicht wie die natürliche Brust aufweisenden Material bestehen und zum Tragen in einem Büstenhalter ohne direkte Befestigung am Körper der Trägerin bestimmt sind, haben auf ihrer Rückseite eine muldenförmige Aushöhlung zur Gewichtsverminderung. Die Größe der Aushöhlung und die dadurch erzielte Gewichtsverminderung werden jedoch dadurch begrenzt, daß mit zunehmender Größe der Aushöhlung die Prothese immer stärker dazu neigt, durch ihr Eigengewicht beim Tragen in einem Büstenhalter in der Weise zusammenzusacken, daß der obere Teil nach unten rutscht. Dieser Schwierigkeit kann man dadurch begegnen, daß man einen weniger weichen Kunststoff verwendet, was natürlich den Nachteil hat, daß man sich dadurch von der Weichheit der natürlichen Brust entfernt. Es besteht auch der Vorschlag, den Kunststoffkörper auf der Rückseite der Prothese, insbesondere den die Aushöhlung umgebenden Teil, aus härter eingestelltem Material auszubilden. Durch diese Maßnahme wird aber die Herstellung der Prothese schwieriger.

Ein gemeinsamer Nachteil aller Brustprothesen, die aus einem in der Konsistenz und dem spezifischen Gewicht der natürlichen Brust entsprechenden Kunststoff bestehen, liegt darin, daß der hierfür in Frage kommende Kunststoff, insbesondere Silikonkautschuk, unverhältnismäßig teuer ist, so daß auch der Preis dieser Prothesen verhältnismäßig hoch ist.

Eine zum Tragen in einem Büstenhalter ohne direkte Befestigung am Körper der Trägerin bestimmte Brustprothese, die der Trägerin das Gefühl vermittelt, daß die Prothese wie die natürliche Brust Gewicht hat, ist aus der GB-A- 1 110 479 bekannt. Bei dieser Brustprothese weist der Prothesenkörper auf seiner Rückseite eine Vertiefung auf, die eine der Vorderseite des Prothesenkörpers folgende Kontur hat. Die Vertiefung ist mit einem aus lose aneinanderliegenden Schnitzeln eines aus Schaumstoff bestehenden Polstermaterials gefüllt, die von einer die Vorderseite des Prothesenkörpers umgebenden und die Rückseite der Prothese bildenden, geschlossenen Hülle aus Stoff in der Vertiefung fest gehalten werden. Die geschlossene Hülle verhindert jedoch den Zugang zu den Schaumstoffschnitzeln, so daß eine nachträgliche Umordnung der Schaumstoffschnitzel zur Anpassung der Prothese an die Form der jeweils entfernten Brust oder eine Entnahme der Schaumstoffschnitzel zur Reinigung oder zum Austausch ohne Entfernung oder Aufschneiden der Hülle nicht möglich ist. Die Reinigung oder der Austausch des Polstermaterials wird auch dadurch erschwert, daß es nicht aus einem einstückigen Körper besteht, sondern unzusammenhängend ist.

Eine Brustprothese, bei der ein Polsterkörper aus Schaumstoff in einen Prothesenkörper zu dessen Stützung eingesetzt ist, ist aus der US-A-3 366 975 bekannt. Diese Brustprothese ist jedoch zum Implantieren bestimmt und hat einen Prothesenkörper, der hohl und vollständig geschlossen ist, und der aufgrund seiner geringen Wanddicke den Charakter einer Hülle ohne eigene Formstabilität hat.

Ferner ist aus der US-A-3 811 133 eine Brustprothese bekannt, bei der ein Polsterkörper aus Watte verwendet wird, der in einem vom Prothesenkörper selbst geschlossenen Hohlraum angeordnet ist. Der Prothesenkörper ist so dünn und so weich, daß er ohne Polsterkörper unter seinem Eigengewicht zusammensacken würde. Der Prothesenkörper und der Polsterkörper sind so leicht, daß ein separates Gewicht auf der Innenseite der Rückwand des Prothesenkörpers erforderlich ist, um die Prothese gewichtsmäßig an die natürliche Brust anzupassen.

Aus der US-A-3 641 592 ist eine Brustprothese bekannt, bei der ein Polsterkörper aus Watte verwendet wird, der in den Hohlraum einer an die Form der natürlichen Brust angepaßten Stoffhülle eingeschlossen ist, die auf ihrer Rückseite eine schlitzartige Öffnung zur Entnahme des Polsterkörpers hat, die durch das Überlappen zweier streifenförmiger Enden der Stoffhülle verschließbar ist. Diese Brustprothese ist auch dafür bestimmt, in einem Büstenhalter getragen und von diesem am Körper gehalten zu werden. Sie ist jedoch wesentlich leichter als die natürliche Brust und sie weicht auch erheblich in den Schwingungs- und Formerhaltungseigenschaften von denen der natürlichen Brust ab.

Die Aufgabe der Erfindung besteht darin, die gattungsgemäße Brustprothese so auszubilden, daß sie beim Tragen in einem Büstenhalter ohne direkte Befestigung am Körper der Trägerin der Trägerin nicht das Gefühl vermittelt, die Prothese sei zu schwer, wobei Material für den Prothesenkörper eingespart wird und sichergestellt ist, daß sie beim Tragen nicht zusammensackt.

Die Aufgabe der Erfindung wird mit den Merkmalen des kennzeichnenden Teiles des Patentanspruches 1 gelöst.

Die erfindungsgemäße Brustprothese hat den Vorteil, daß die Vertiefung wesentlich größer gemacht werden kann, so daß zur Herstellung des Prothesenkörpers wesentlich weniger Kunststoff verbraucht wird, ohne daß die Prothese dabei ihre Stabilität einbüßt, denn der Polsterkörper verhindert das Zusammensacken des Prothesenkörpers. Ein weiterer Vorteil besteht darin, daß der Polsterkörper aus der Vertiefung zur Reinigung leicht entnommen und ohne weiteres in sie wieder eingesetzt oder gegen einen neuen ausgetauscht werden kann, wobei im zusammengesetzten Zustand der Prothese durch die Stoffhülle ein guter Zusammenhalt zwischen dem Prothesenkörper und dem Polsterkörper erzielt wird.

Die Kunststoffmasse des Prothesenkörpers kann mit einer Folie überzogen sein, da die Kunststoffe mit einer geeigneten Weichheit üblicherweise zum Kleben neigen. Bevorzugt wird für die Kunststoffmasse des Prothesenkörpers Silikonkautschuk und für die Folie Polyurethan.

Der Polsterkörper kann aus Schaumstoff bestehen, wie z. B. aus einem geschäumten Polyurethanformstück. Es ist aber auch möglich, daß der Polsterkörper aus Fasern, beispielsweise aus einem Wattebausch, besteht. Der aus Fasern bestehende Polsterkörper kann von einem Stoffbeutel umgeben sein.

Vorzugsweise ist die Öffnung der Stoffhülle auf deren Rückseite angeordnet, schlitzförmig ausgebildet und durch zwei sich überlappende Stoffenden verschließbar. Durch diese Öffnung kann der Polsterkörper und ggf. auch der Prothesenkörper ohne weiteres entnommen werden.

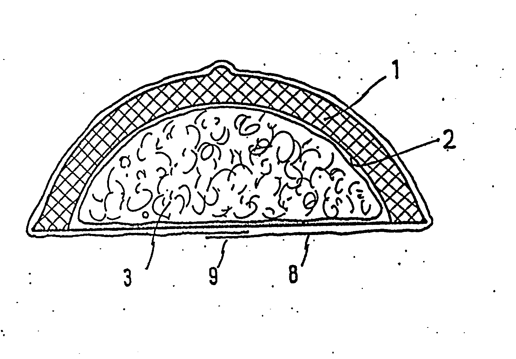

Ein Ausführungsbeispiel der Erfindung ist in der Zeichnung dargestellt und wird im folgenden näher beschrieben, wobei die Zeichnung einen Querschnitt durch eine Brustprothese nach der Erfindung zeigt.

Die in der Zeichnung dargestellte Brustprothese weist einen Prothesenkörper 1 aus Silikonkautschuk auf, der eine der Form der natürlichen Brust entsprechende Vorderseite und eine Vertiefung 2 auf seiner Rückseite hat. Wie aus der Zeichnung ersichtlich ist, hat die Vertiefung 2 eine der Vorderseite des Prothesenkörpers 1 im wesentlichen folgende Kontur. In der Vertiefung 2 ist ein Polsterkörper 3 in Form eines Wattebausches angeordnet, welcher ein Zusammensacken des Prothesenkörpers 1 beim Tragen der Prothese in einem Büstenhalter verhindert. Der Prothesenkörper 1 und Polsterkörper 3 sind zusammen in eine Stoffhülle 8 eingeschlossen, welche, wie aus er Zeichnung ersichtlich, an der Vorderseite des Prothesenkörpers 1 und an der Rückseite des Polsterkörpers 3 anliegt, wodurch der Polsterkörper 3 in der Vertiefung 2 gehalten wird. Die Stoffhülle 8 hat auf ihrer Rückseite eine schlitzartige Öffnung 9, in deren Bereich sich zwei Stoffenden überlappen. Durch Auseinanderbewegen der beiden sich überlappenden Stoffenden der Stoffhülle 8 wird die Stoffhülle 8 geöffnet, so daß der Polsterkörper 2 und der Prothesenkörper 1 aus ihr entnommen und in sie eingesetzt werden können.

## Patentansprüche

1. Brustprothese mit einem weichen, elastischen Prothesenkörper (1), der eine der Form der natürlichen Brust entsprechende Vorderseite und eine eine Vertiefung (2) aufweisende Rückseite hat und im wesentlichen aus einer Kunststoffmasse besteht, die in ihrer Konsistenz und in ihrem spezifischen Gewicht etwa der natürlichen Brust entspricht, und einem Polsterkörper (3), der in der Vertiefung (2) des Prothesenkörpers (1) angeordnet und in ihr gehalten ist und ein geringeres spezifisches Gewicht als die Kunststoffmasse des Prothesenkörpers hat, dadurch gekennzeichnet, daß die Vertiefung (2) eine der Vorderseite des Prothesenkörpers (1) im wesentlichen folgende Kontur hat und daß der Polsterkörper (3) durch eine an der Vorderseite des Prothesenkörpers (1) und an der Rückseite des Polsterkörpers (3) anliegende Stoffhülle (8) in der Vertiefung (2) des Prothesenkörpers (1) lösbar gehalten ist, die eine verschließbare Öffnung (9) aufweist, durch die der Polsterkörper (3) in die Vertiefung (2) des Prothesenkörpers (1) einführbar und aus ihr entnehmbar ist.

2. Brustprothese nach Anspruch 1, dadurch gekennzeichnet, daß die Kunststoffmasse des Prothesenkörpers (1) mit einer Folie überzogen ist.

3. Brustprothese nach Anspruch 2, dadurch gekennzeichnet, daß die Kunststoffmasse des Prothesenkörpers (1) aus Silikonkautschuk und die Folie aus Polyurethan besteht.

4. Brustprothese nach einem der vorhergehenden Ansprüche dadurch gekennzeichnet, daß der Polsterkörper (3) aus Schaumstoff besteht.

5. Brustprothese nach einem der Ansprüche 1 bis 3, dadurch gekennzeichnet, daß der Polsterkörper (3) aus Fasern besteht.

6. Brustprothese nach Anspruch 5, dadurch gekennzeichnet, daß der aus Fasern bestehende Polsterkörper (3) von einem Stoffbeutel umgeben ist.

7. Brustprothese nach Anspruch 5 oder 6, dadurch gekennzeichnet, daß der Polsterkörper (3) ein Wattebausch ist.

8. Brustprothese nach einem der vorhergehenden Ansprüche, dadurch gekennzeichnet, daß die Öffnung (9) der Stoffhülle (8) auf deren Rückseite angeordnet, schlitzförmig ausgebildet und durch zwei sich überlappende Stoffenden verschließbar ist.

## Claims

1. A mastoprosthesis comprising a soft resilient prosthesis body (1), which has a front surface conforming to the shape of the natural breast and a rear surface presenting a depression and consists substantially of a synthetic material resembling in its consistency and specific weight the natural breast, and a pad member (3) which is disposed in the depression (2) of the prosthesis body (1) and retained therein and lighter in specific weight than the synthetic material of the prosthesis body, characterized in that the depression (2) is contoured to conform substantially to the contour of the front surface of the prosthesis body (1) and that the pad member (3) is retained detachably in the depression (2) of the prosthesis body (1) by a fabric enclosure (8) which abuts the front surface of the prosthesis body (1) and the rear surface of the pad member (3), said fabric enclosure having an opening (9) which is adapted to be closed and through which the pad member (3) can be inserted into the depression (2) of the prosthesis body (1) and removed from it.

2. A mastoprosthesis according to claim 1, characterized in that the synthetic material of the prosthesis body (1) is covered by a film.

3. A mastoprosthesis according to claim 2, characterized in that the synthetic material of the prosthesis body (1) consists of silicone rubber and the film consists of polyurethane.

4. A mastoprosthesis according to anyone of the preceding claims, characterized in that the pad member (3) consists of foam material.

5. A mastoprosthesis according to anyone of claims 1 to 3, characterized in that the pad member (3) consists of fibres.

6. A mastoprosthesis according to claim 5, characterized in that the pad member (3) consisting of fibres is surrounded by a fabric poach.

7. A mastoprosthesis according to claim 5 or 6, characterized in that the pad member (3) is a wad member.

8. A mastoprosthesis according to anyone of the preceding claims, characterized in that the opening (9) of the fabric enclosure (8) is disposed at its rear surface, adapted to be slot-shaped, and can be closed by two overlying fabric ends.

## Revendications

1. Prothèse mammaire avec un corps de prothèse (1) élastique et souple, qui présente une face avant, correspondant à la forme du sein naturel, un évidement (2) sur sa face arrière, et qui se compose essentiellement d'une matière plastique, dont la consistance et le poids spécifique correspondent à peu près à ceux du sein naturel, et avec un corps de rembourrage (3), logé et maintenu dans l'évidement (2) du corps de prothèse (1), et d'un poids spécifique inférieur à celui de la masse plastique du corps précité, caractérisé en ce que le contour de l'évidement (2) correspond essentiellement à la face avant du corps de prothèse (1), et en ce que le corps de rembourrage (3), amovible, est maintenu dans l'évidement (2) du corps de prothèse (1) par une gaine de tissu (8), qui s'applique sur sa face arrière et sur la face avant du corps de prothèse (1), cette gaine présentant une ouverture (9) obturable, qui permet d'introduire le corps de rembourrage (3) dans l'évidement (2) du corps de prothèse (1), et de l'en retirer.

2. Prothèse mammaire suivant la revendication 1, caractérisée en ce que la masse plastique du corps de prothèse (1) est revêtue d'une feuille.

3. Prothèse mammaire suivant la revendication 2, caractérisée en ce que la masse plastique du corps de prothèse (1) est en caoutchouc au silicone et la feuille en polyuréthane.

4. Prothèse mammaire suivant l'une quelconque des revendications précédentes, caractérisée en ce que le corps de rembourrage (3) est en mousse.

5. Prothèse mammaire suivant l'une quelconque des revendications 1 à 3, caractérisée en ce que le corps de rembourrage (3) est en fibres.

6. Prothèse mammaire suivant la revendication 5, caractérisée en que le corps de rembourrage (3), composé de fibres, est enveloppé par un sac de tissu.

7. Prothèse mammaire suivant l'une des revendications 5 et 6, caractérisée en ce que le corps de rembourrage (3) est un tampon d'ouate.

8. Prothèse mammaire suivant l'une quelconque des revendications précédentes, caractérisée en ce que l'ouverture (9) de la gaine de tissu (8), disposée sur la face arrière de cette dernière, est représentée par une fente et peut être fermée par le chevauchement de deux bouts de tissu.